# EUROPEAN PATENT APPLICATION

(11) **EP 4 156 093 A1**
(43) Date of publication of application: **29.03.2023**
(21) Application number: 21833962.0
(22) Date of filing: 14.06.2021
(51) Int. Cl.: G06T 7/00, G01N 33/48, G01N 33/483, G06N 20/00

(54) **ASSESSMENT SUPPORT DEVICE, INFORMATION PROCESSING DEVICE, AND LEARNING METHOD**

(30) Priority: 30.06.2020 JP 2020113403
(71) Applicant: Sony Group Corporation, Tokyo 108-0075 (JP)
(72) Inventor: TERAMOTO, Toya, Tokyo 108-0075 (JP); AISAKA, Kazuki, Tokyo 108-0075 (JP); ONO, Yuki, Tokyo 108-0075 (JP)
(74) Representative: D Young & Co LLP
(86) International application number: PCT/JP2021/022454
(87) International publication number: WO 2022/004337

(57) **Abstract**

Making it possible to improve accuracy of machine learning. A determination support device includes: a derivation unit (100) that derives an estimation result of determination on a first pathological image obtained by imaging a biological sample, the derivation being performed using a multi-stage trained model in which class classification can be set in each stage.

## Description

### Field

The present disclosure relates to a determination support device, an information processing device, and a training method.

### Background

In recent years, there has been a technology developed for supporting diagnosis by a clinician or the like by outputting a diagnostic estimation result obtained by a learning model from a medical image which is a pathological image or the like.

### Citation List

### Non Patent Literature

Non Patent Literature 1: Yun Liu, Krishna Gadepalli, Mohammad Norouzi, George E. Dahl, Timo Kohlberger, Aleksey Boyko, Subhashini Venugopalan, Aleksei Timofeev, Philip Q. Nelson, Gregory S. Corrado, Jason D. Hipp, Lily Peng, Martin C. Stumpe: Detecting Cancer Metastases on Gigapixel Pathology Images. CoRR abs/1703.02442 (2017)
Non Patent Literature 2: Ezgi Mercan, Sachin Mehta, Jamen Bartlett, Linda G. Shapiro, Donald L. Weaver, and Joann G. Elmore: Assessment of Machine Learning of Breast Pathology Structures for Automated Differentiation of Breast Cancer and High-Risk Proliferative Lesions. JAMA Network Open, 2(8):e198777-e198777, 08 2019.

### Summary

### Technical Problem

However, even though the work of annotating a pathological image to create training data is a complicated work requiring much labor and time in known cases, a large amount of training data has been needed to improve accuracy of machine learning.

In view of this, the present disclosure proposes a determination support device, an information processing device, and a training method that enable improvement in accuracy of machine learning.

### Solution to Problem

To solve the above-described problem, a determination support device according to one aspect of the present disclosure comprises a derivation unit that derives an estimation result of determination on a first pathological image obtained by imaging a biological sample, the derivation being performed using a multi-stage trained model in which class classification can be set in each stage.

### Brief Description of Drawings

FIG. 1 is a diagram illustrating a diagnosis support system according to an embodiment.
FIG. 2 is a diagram illustrating imaging processing according to an embodiment.
FIG. 3 is a diagram illustrating imaging processing according to an embodiment.
FIG. 4 is a diagram illustrating processing of generating a partial image (tiled image).
FIG. 5 is a diagram illustrating a pathological image according to an embodiment.
FIG. 6 is a diagram illustrating a pathological image according to an embodiment.
FIG. 7 is a diagram illustrating an example of a viewing mode by a viewer of a pathological image.
FIG. 8 is a diagram illustrating an example of a viewing history storage unit included in a server.
FIG. 9 is a diagram illustrating an example of a derivation device and a display control device according to an embodiment.
FIG. 10 is a schematic diagram illustrating a flow when creating training data according to an embodiment.
FIG. 11 is a screen view illustrating an example of a user interface for creating training data according to an embodiment.
FIG. 12 is a hierarchical diagram illustrating an example of a hierarchical structure of a pathological case to be diagnosed.
FIG. 13 is a schematic diagram illustrating an example of a stepwise trained model creation flow (AI creation flow) according to an embodiment.
FIG. 14 is a diagram illustrating an annotation function according to a first example of an embodiment.
FIG. 15 is a diagram illustrating an annotation function according to a second example of an embodiment.
FIG. 16 is a diagram illustrating an annotation function according to a third example of an embodiment.
FIG. 17 is a diagram illustrating a similarity search function with annotation according to an embodiment.
FIG. 18 is a diagram illustrating a user interface according to a first display example of an embodiment.
FIG. 19 is a diagram illustrating a user interface according to a second display example of an embodiment (part 1).
FIG. 20 is a diagram illustrating a user interface according to the second display example of an embodiment (part 2).
FIG. 21 is a diagram illustrating an example of an AI creation flow when a diagnosis support system according to an embodiment is applied to diagnosis for lymph node metastasis of breast cancer.
FIG. 22 is a hardware configuration diagram illustrating an example of a computer that implements functions of a derivation device.

### Description of Embodiments

Embodiments of the present disclosure will be described below in detail with reference to the drawings. In each of the following embodiments, the same parts are denoted by the same reference symbols, and a repetitive description thereof will be omitted.

The present disclosure will be described in the following order.
1. Embodiment
   1-1. System configuration
   1-2. Various types of information
      1-2-1. Pathological image
      1-2-2. Viewing history information
   1-3. Derivation device
   1-4. Creating training data
      1-4-1. Flow of creating training data
      1-4-2. User interface
   1-5. Creating trained model by multi-stage training step
      1-5-1. Hierarchical representation of pathological cases
      1-5-2. Creating stepwise trained model
         1-5-2-1. Stepwise creation flow
   1-6. Display with varied magnification and granularity
      1-6-1. First example
      1-6-2. Second example
      1-6-3. Third example
   1-7. Similarity search function with annotation
   1-8. Display example of inference result
      1-8-1. First display example
      1-8-2. Second display example
   1-9. Another example of hierarchical structure
   1-10. Action and effects
2. Other embodiments
   2-1. Display device
   2-2. Imaging device
   2-3. Server
   2-4. Pathological image
   2-5. Hardware configuration

### (1. Embodiment)

### [1-1. System configuration]

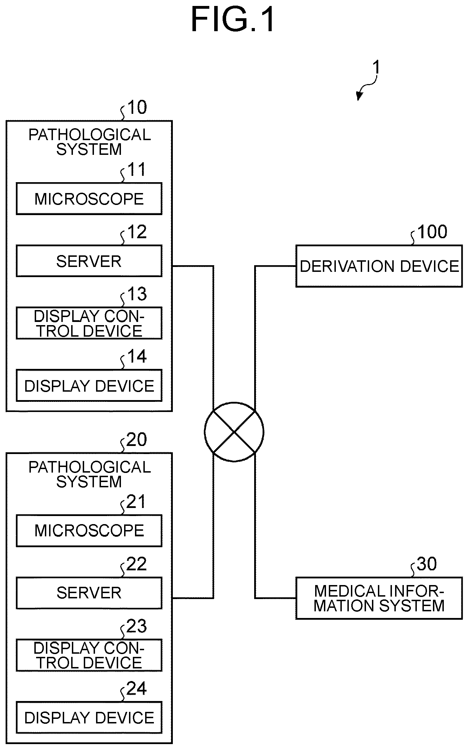

First, a diagnosis support system (determination support system, information processing system, and information processing device) according to an embodiment will be described with reference to FIG. 1. Note that "diagnosis" in the present description can also be interpreted as "determination" in a broader sense. FIG. 1 is a diagram illustrating a diagnosis support system according to the present embodiment. As illustrated in FIG. 1, a diagnosis support system 1 includes a pathology system 10, a pathology system 20, a medical information system 30, and a derivation device 100.

The pathology system 10 is a system mainly used by a pathologist, and is applied to a laboratory and a hospital, for example. As illustrated in FIG. 1, the pathology system 10 includes a microscope 11, a server 12, a display control device 13, and a display device 14.

The microscope 11 is an imaging device that has a function of an optical microscope, captures an image of a specimen to be observed placed on a glass slide, and acquires a pathological image (an example of a medical image) that is a digital image.

Here, the specimen to be observed may be prepared for the purpose of pathological diagnosis or the like from a biologically originated specimen (hereinafter, referred to as a biological specimen) such as a body sample or a tissue sample collected from a human body. The specimen may be a tissue section, a cell, or a fine particle, and there is no particular limitation regarding details of the specimen, such as the type of tissue (for example, organ or the like) used, the type of disease to be targeted, attributes of the subject (for example, age, sex, blood type, race, and the like), or the lifestyle of the subject (for example, dietary habits, exercise habits, and smoking habits). Incidentally, a tissue section can include, for example, a section before staining of a tissue section to be stained (hereinafter, it is also simply referred to as a section), a section adjacent to the stained section, a section different from the stained section in a same block (sampled from the same location as the stained section), a section in a different block in a same tissue (sampled from a different place from the stained section), and a section collected from a different patient.

The server 12 is a device that stores and preserves a pathological image captured by the microscope 11 in a storage unit (not illustrated). When having received a viewing request from the display control device 13, the server 12 retrieves a pathological image from a storage unit (not illustrated) and transmits the retrieved pathological image to the display control device 13.

The display control device 13 transmits a request for viewing a pathological image received from a user such as a clinician or a pathologist to the server 12. The display control device 13 then controls the display device 14 to display a pathological image received from the server 12.

The display device 14 has a screen using liquid crystal, electro-luminescence (EL), and cathode ray tube (CRT), for example. The display device 14 may be a device achieving 4K or 8K resolution, or may be formed by a plurality of display devices. The display device 14 displays the pathological image controlled to be displayed by the display control device 13. In addition, the server 12 stores the viewing history information related to a region of the pathological image observed by the pathologist via the display device 14. The viewing history information may be, for example, information related to a viewing history of a pathological image acquired by a user such as a clinician or a pathologist in a past case.

The pathology system 20 is a system applied to a hospital different from the hospital having the pathology system 10. The pathology system 20 includes a microscope 21, a server 22, a display control device 23, and a display device 24. The components included in the pathology system 20 are similar to those of the pathology system 10, and thus description thereof is omitted.

The medical information system 30 is a system that stores information related to diagnosis of patients. For example, when it is difficult to diagnose a state of the disease only from an image in an endoscopic examination or the like in a predetermined hospital, a biopsy may be performed to perform a definite diagnosis by pathological diagnosis. A specimen prepared from tissue collected from a patient is imaged by the microscope 11 of the pathology system 10, and a pathological image obtained by imaging is stored in the server 12. The display control device 13 controls to display the pathological image on the display device 14, with which pathological diagnosis is performed by a user such as a pathologist using the pathology system 10. Note that the user who uses the pathology system 10 is not limited to a pathologist or the like who uses the pathology system 10 for the purpose of diagnosis or the like, and may include a pathologist or the like who uses the pathology system 10 for research purposes. That is, the diagnosis support system 1 according to the present embodiment can be used not only for the purpose of pathological diagnosis but also for research purposes. Hereinafter, for simplification, a person who uses the pathology system 10 is referred to as a clinician, a pathologist, or a user. The clinician performs a definite diagnosis based on a pathological diagnosis result, and a result of the definite diagnosis is stored in the medical information system 30. The medical information system 30 stores information related to diagnosis, such as information for identifying a patient, patient disease information, examination information and image information used for diagnosis, a diagnosis result, and prescription medicine. Note that the medical information system 30 is referred to as an electronic medical record system or the like.

Incidentally, the accuracy of pathological diagnosis varies for each pathologist. Specifically, the diagnosis result by the pathological image can vary for each pathologist depending on the years of experience and expertise of the pathologist. For this reason, it is desired to derive diagnosis support information, which is information for supporting diagnosis using machine learning, for the purpose of supporting pathological diagnosis.

As the diagnosis support using machine learning, for example, it is conceivable to derive a diagnostic estimation result for a pathological image by using a process including: annotating a pathological image acquired in the past case with a diagnosis result for a past disease case given by a clinician, a pathologist, or the like; training a learning model using the annotated pathological image as training data; and inputting a pathological image to be newly diagnosed to a trained learning model (hereinafter, referred to as a trained model).

However, as described above, even though the work of annotating a pathological image to create training data is a complicated work requiring much labor and time in known cases, a large amount of training data has been needed to improve the accuracy of machine learning.

In view of this situation, the present embodiment makes it possible to easily annotate an image obtained by imaging a biological sample including a biological tissue, such as a pathological image (hereinafter, referred to as a pathological image for simplicity). This makes it possible to easily improve the accuracy of machine learning, enabling the user such as a clinician or a pathologist to perform diagnosis on a case with higher accuracy.

In the present embodiment, the pathological image used as training data may be a pathological image included in a tiled image group having a pyramid structure created for each case. Although details of the tiled image group having the pyramid structure will be described below, the tiled image group is schematically an image group including pathological images with higher magnification and higher resolution in lower layers. In the same case, the image group of each layer represents one identical specimen as a whole. In the present description, an entire image including an image group of the lowermost layer, in other words, the highest magnification is referred to as an entire image.

An example of processing performed by the derivation device 100 will be described with reference to the example of FIG. 1. In the example of FIG. 1, it is assumed that diagnosis-related information from a pathologist is accumulated daily in the server 12 of the pathology system 10. That is, the server 12 stores a pathological image (second pathological image) corresponding to a first affected tissue. In addition, in the example of FIG. 1, it is assumed that the derivation device 100 provides diagnosis support information to the pathology system 20.

First, the derivation device 100 acquires daily accumulated pathological images from the server 12 of the pathology system 10. Furthermore, the derivation device 100 causes the user to annotate the acquired pathological image with a diagnosis result. The derivation device 100 then acquires the annotated pathological image from the medical information system 30. The derivation device 100 trains the learning model using the acquired annotated pathological image as training data to create a trained model for estimating a diagnosis result from a pathological image (first pathological image) corresponding to a second affected tissue different from the first affected tissue.

Consequently, it is assumed that a pathological image corresponding to the second affected tissue has been generated by the microscope 21 in the pathology system 20. At this time, when having received a request to display a pathological image from a user such as a clinician or a pathologist, the display control device 23 transmits the pathological image to the derivation device 100. The derivation device 100 uses the trained model to derive a diagnostic estimation result for the case from the pathological image (derivation unit), and outputs the derived estimation result to the display control device 23 as a part of diagnosis support information.

As described above, the derivation device 100 may specify the entire image of the tiled image group including a basis image and may specify the viewing history information related to the tiled image group of the entire image, and may output the specified entire image and viewing history information to the display control device 23 as a part of the diagnosis support information.

Although the above is an example of training the learning model using the pathological image stored in the server 12 of the pathology system 10 as training data, the derivation device 100 may train the learning model using the pathological image stored in the server 22 of the pathology system 20 as training data, or may train the learning model using both the pathological image stored in the server 12 and the pathological image stored in the server 22 as the training data. In addition, while the above description is an example in which the derivation device 100 provides diagnosis support information to the display control device 23, the derivation device 100 may provide the diagnosis support information to the display control device 13.

Furthermore, although the pathology system 10 and the pathology system 20 have been described as separate from each other, the pathology system 10 and the pathology system 20 may be the same system. More specifically, the diagnosis support system 1 may include the pathology system 10 alone. In this case, the derivation device 100 trains the learning model using the pathological image stored in the server 12 as training data, and provides diagnosis support information to the display control device 13 in response to a request from the display control device 13. In addition, the number of pathology systems included in the diagnosis support system 1 may be three or more. In this case, the derivation device 100 may collect pathological images accumulated in each pathology system to create training data, and may train the learning model using the training data. Furthermore, in the above example, the medical information system 30 may be the same system as the pathology system 10 or 20. That is, the annotated pathological image may be stored in the server 12 or 22.

Note that the derivation device 100 according to the present embodiment may be implemented by a server, a cloud server, or the like arranged on a network, or may be implemented by a server 12/22 arranged in the pathology system 10/20. Alternatively, a part of the derivation device 100 (for example, a pathological image acquisition unit 131, a training data acquisition unit 132, and a training unit 133 in a control unit 130, a storage unit 120, and the like) may be implemented by a server, a cloud server, or the like arranged on a network, while the remaining part (for example, a derivation unit 134 and the like) may be implemented by the server 12/22 of the pathology system 10/20, that is, the components may be implemented by using distributed arrangement on a system constructed via a network.

The diagnosis support system 1 has been briefly described above. Hereinafter, in the description of the configuration and processing of each device which will be given in detail, various types of information as a premise of the description (data structure of a pathological image, viewing history information regarding a pathological image, and annotated pathological image) will be first described. Note that the following description is an example in which the derivation device 100 trains a learning model using training data accumulated in the pathology system 10 and provides diagnosis support information to the pathology system 20.

### [1-2. Various types of information]

### [1-2-1. Pathological image]

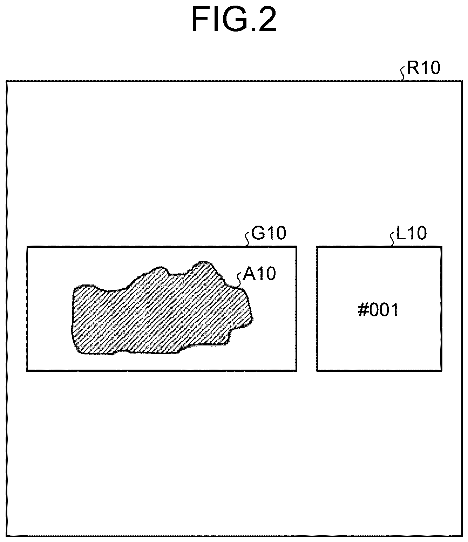

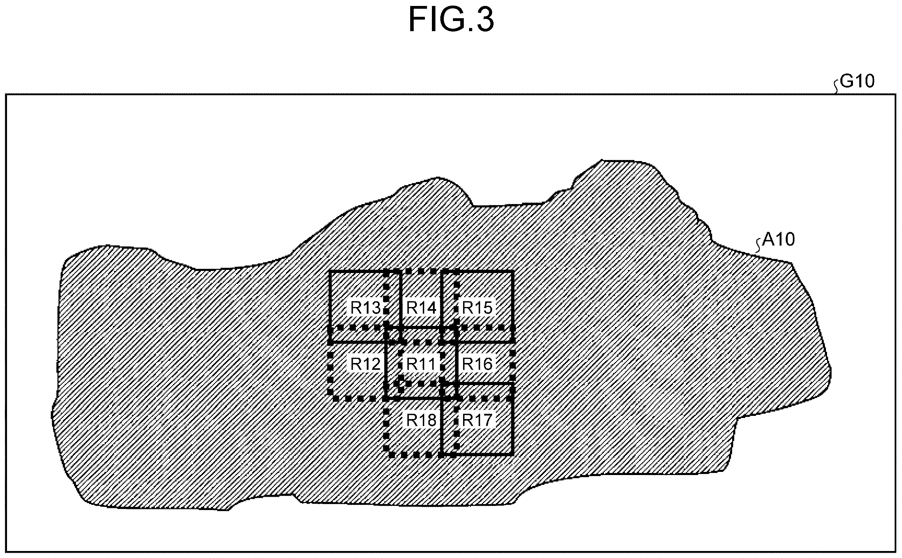

As described above, a pathological image is generated by imaging a specimen using the microscope 11 or the microscope 21. First, imaging processing using the microscope 11 and the microscope 21 will be described with reference to FIGS. 2 and 3. FIGS. 2 and 3 are diagrams illustrating imaging processing according to the present embodiment. Since the microscope 11 and the microscope 21 perform similar imaging processing, the microscope 11 will be described here as an example. The microscope 11 described below includes a low-resolution imaging unit for imaging at low resolution and a high-resolution imaging unit for imaging at high resolution.

In FIG. 2, the region that can be imaged by the microscope 11, namely, an imaging region R10, includes a glass slide G10 on which a specimen A10 is mounted. The glass slide G10 is placed on a stage (not illustrated), for example. The microscope 11 images the imaging region R10 by the low-resolution imaging unit to generate an entire image, which is a pathological image of the entire specimen A10. Label information L10 illustrated in FIG. 2 includes description of identification information (for example, a character string or a QR code (registered trademark)) for identifying the specimen A10. By associating the identification information described as the label information L10 with a patient, it is possible to specify the patient corresponding to the entire image. In the example of FIG. 2, "#001" is described as identification information. The label information L10 may also include a simple description of the specimen A10.

Subsequently, after generating the entire image, the microscope 11 specifies a region where the specimen A10 exists from the entire image, and uses the high-resolution imaging unit to sequentially image each divided region, obtained by dividing the region where the specimen A10 exists, in each predetermined size. For example, as illustrated in FIG. 3, the microscope 11 first images a region R11, and generates a high-resolution image I11 that is an image representing a partial region of the specimen A10. Subsequently, the microscope 11 moves the stage to image a region R12 by the high-resolution imaging unit, and generates a high-resolution image I12 corresponding to the region R12. Similarly, the microscope 11 generates high-resolution images I13, I14,··· corresponding to regions R13, R14,··· . Although FIG. 3 only illustrates regions up to the region R18, the microscope 11 sequentially moves the stage to image all divided regions corresponding to the specimen A10 by the high-resolution imaging unit, and generates a high-resolution image corresponding to each divided region.

Meanwhile, the glass slide G10 might accidentally move on the stage when the state is moved. Movement of the glass slide G10 might cause generation of a region having no captured image of the specimen A10. As illustrated in FIG. 3, the microscope 11 performs imaging by the high-resolution imaging unit such that adjacent divided regions partially overlap each other, making it possible to prevent the occurrence of the region having no captured image even when the glass slide G10 moves.

Note that the low-resolution imaging unit and the high-resolution imaging unit described above may include different optical systems or the same optical system. When the optical system is the same, the microscope 11 changes the resolution according to the imaging target. Although the above description is an example in which the imaging region is changed by moving the stage, the imaging region may be changed by moving the optical system (high-resolution imaging unit or the like) by the microscope 11. Furthermore, FIG. 3 illustrates an example in which the microscope 11 captures an image starting from the central portion of the specimen A10. However, the microscope 11 may image the specimen A10 in an order different from the imaging order illustrated in FIG. 3. For example, the microscope 11 may capture an image starting from the outer peripheral portion of the specimen A10. Furthermore, the above is an example in which the high-resolution imaging unit images only the region where the specimen A10 exists. However, since there is a case where it is difficult to accurately detect the region where the specimen A10 exists, the microscope 11 may divide the entire region of the imaging region R10 or the glass slide G10 illustrated in FIG. 2 and perform imaging with the high-resolution imaging unit.

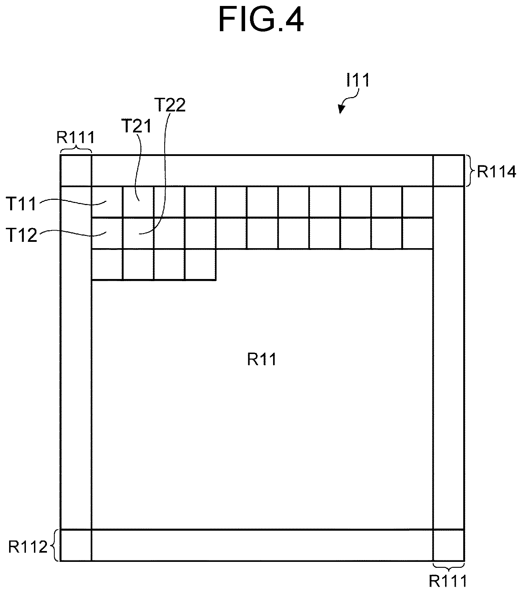

Subsequently, each high-resolution image generated by the microscope 11 is divided into a predetermined size. This generates a partial image (hereinafter, referred to as a tiled image) from the high-resolution image. This point will be described with reference to FIG. 4. FIG. 4 is a diagram illustrating processing of generating a partial image (tiled image). FIG. 4 illustrates the high-resolution image I11 corresponding to the region R11 illustrated in FIG. 3. Note that the following description assumes that a partial image is generated from a high-resolution image by the server 12. However, the partial image may be generated by a device (for example, an information processing device mounted inside the microscope 11, or the like) other than the server 12.

In the example illustrated in FIG. 4, the server 12 generates 100 tiled images T11, T12,··· by dividing one high-resolution image I11. For example, when the high-resolution image I11 has a resolution 2560 × 2560 [pixels], the server 12 generates, from the high-resolution image I11, hundred tiled images T11, T12,··· each of which having a resolution 256 × 256 [pixels]. Similarly, the server 12 generates tiled images by dividing other high-resolution images into similar sizes.

Note that, in the example of FIG. 4, regions R111, R112, R113, and R114 are regions overlapping other adjacent high-resolution images (not illustrated in FIG. 4). The server 12 performs aligning of the overlapping regions by a technique such as template matching to perform stitching processing on the adjacent high-resolution images. In this case, the server 12 may generate the tiled image by dividing the high-resolution image after the stitching processing. Alternatively, the server 12 may generate tiled images of regions other than the regions R111, R112, R113, and R114 before the stitching processing, and then generate tiled images of the regions R111, R112, R113, and R114 after the stitching processing.

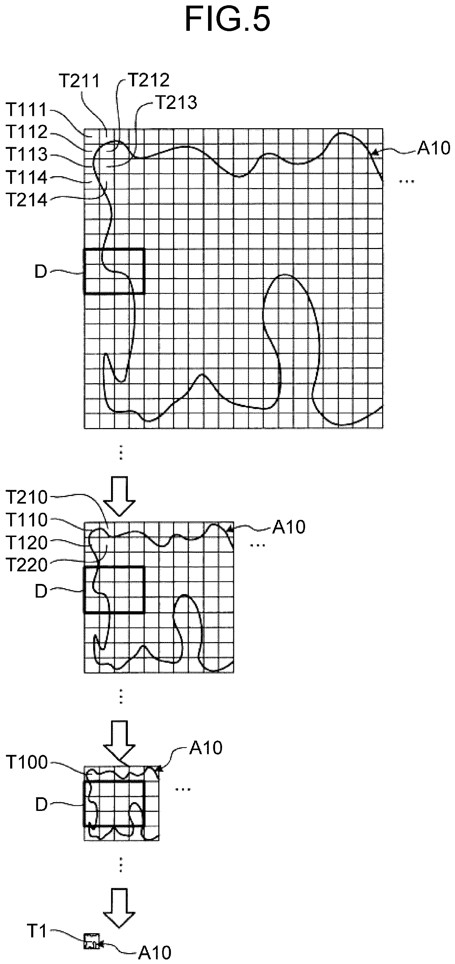

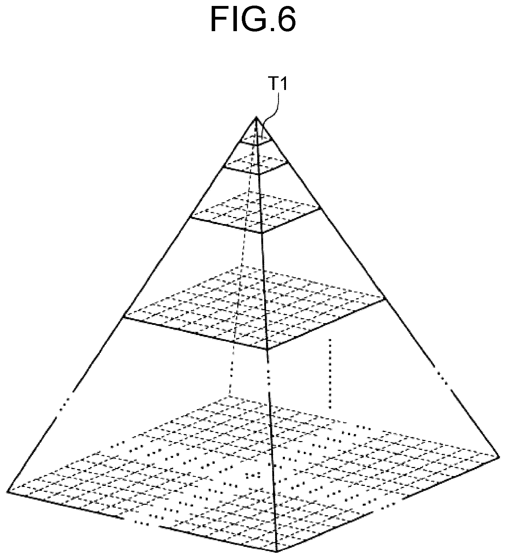

In this manner, the server 12 generates a tiled image that is a minimum unit of the captured image of the specimen A10. The server 12 then sequentially combines the tiled images of the minimum unit to generate tiled images having different hierarchies. Specifically, the server 12 generates one tiled image by combining a predetermined number of adjacent tiled images. This will be described with reference to FIGS. 5 and 6. FIGS. 5 and 6 are diagrams illustrating a pathological image according to the present embodiment.

The upper part of FIG. 5 illustrates a tiled image group of a minimum unit generated from each high-resolution image by the server 12. In the example in the upper part of FIG. 5, by combining four tiled images T111, T112, T211, and T212 adjacent to each other among the tiled images, the server 12 generates one tiled image T110. For example, when the resolution of each of the tiled images T111, T112, T211, and T212 is 256 × 256, the server 12 generates a tiled image T110 having a resolution of 256 × 256. Similarly, the server 12 generates a tiled image T120 by combining four tiled images T113, T114, T213, T214 adjacent to each other. In this manner, the server 12 generates a tiled image obtained by combining a predetermined number of tiled images of the minimum unit.

Furthermore, among the tiled images obtained by combining the tiled images of the minimum unit, the server 12 further combining tiled images adjacent to each other to generate another tiled image. In the example of FIG. 5, the server 12 generates one tiled image T100 by combining four tiled images T110, T120, T210, and T220 adjacent to each other. For example, when the tiled images T110, T120, T210, and T220 each have a resolution 256 × 256, the server 12 generates a tiled image T100 having the resolution 256 × 256. For example, based on a tiled image having a resolution of 512 × 512 obtained by combining four tiled images adjacent to each other, the server 12 applies 4-pixel averaging, a weighting filter (processing of weighting close pixels more than far pixels), 1/2 thinning processing, or the like, on the image to generate an image having a resolution of 256 × 256.

By repeating such combining processing, the server 12 finally generates one tiled image having a resolution similar to the resolution of the tiled image of the minimum unit. For example, when the tiled image of the minimum unit has a resolution 256 × 256 as in the above example, the server 12 repeats the above-described combining processing to generate one tiled image T1 having a final resolution of 256 × 256.

FIG. 6 schematically illustrates the tiled image illustrated in FIG. 5. In the example illustrated in FIG. 6, the lowermost layer tiled image group includes a minimum unit of tiled image generated by the server 12. Furthermore, the tiled image group in the second lowest layer is a tiled image obtained by combining the tiled image groups of the lowermost layer. An uppermost tiled image T1 indicates one tiled image to be finally generated. In this manner, the server 12 generates, as a pathological image, a tiled image group having a hierarchy such as a pyramid structure illustrated in FIG. 6.

Note that a region D illustrated in FIG. 5 is an example of a region displayed on a display screen of the display device 14 or 24. For example, the resolution displayable by the display device, in an assumable case, corresponds to a tiled image of three vertical images and a tiled image of four horizontal images. In this case, as in the region D illustrated in FIG. 5, the level of detail of the specimen A10 displayed on the display device varies depending on the hierarchy to which the tiled image to be displayed belongs. For example, when the tiled image in the lowermost layer is used, a small region in the specimen A10 is displayed in a high level of detail. In addition, as the tiled image goes toward the upper layers, a wider region in the specimen A10 is displayed in a low level of detail.

The server 12 stores tiled images of the individual hierarchies as illustrated in FIG. 6 in a storage unit (not illustrated). For example, the server 12 stores each tiled image together with tile identification information (an example of partial image information) with which each tiled image can be uniquely identified. In this case, when having received a tiled image acquisition request including tile identification information from another device (for example, the display control device 13 or the derivation device 100), the server 12 transmits a tiled image corresponding to the tile identification information to the another device. Furthermore, for example, the server 12 may store each tiled image together with hierarchy identification information for identifying each hierarchy and tile identification information with which tiles can be uniquely identified in a same hierarchy. In this case, when the server 12 has received a tiled image acquisition request including hierarchy identification information and tile identification information from the another device, the server 12 transmits, to the another device, a tiled image corresponding to the tile identification information among the tiled images belonging to the hierarchy corresponding to the hierarchy identification information.

Note that the server 12 may store the tiled images of the respective hierarchies as illustrated in FIG. 6 in a storage device other than the server 12. For example, the server 12 may store tiled images of each hierarchy in a cloud server or the like. Furthermore, the processing of generating tiled images illustrated in FIGS. 5 and 6 may be executed by a cloud server or the like.

Furthermore, the server 12 need not store the tiled images of all the hierarchies. For example, the server 12 may store only the tiled image of the lowermost layer, may store only the tiled image of the lowermost layer and the tiled image of the uppermost layer, or may store only the tiled image of a predetermined hierarchy (for example, odd-numbered hierarchies, even-numbered hierarchies, and the like.). At this time, when the tiled image requested from another device is not stored in the server 12, the server 12 generates a tiled image requested from the another device by dynamically combining the stored tiled images. In this manner, by thinning out the tiled images to be stored, the server 12 can prevent the storage capacity constraint.

Furthermore, although the imaging conditions are not mentioned in the above example, the server 12 may store the tiled images of the respective hierarchies as illustrated in FIG. 6 for each imaging condition. Examples of the imaging condition include a focal length with respect to a subject (such as the specimen A10). For example, the microscope 11 may capture images of the same subject while changing the focal length. In this case, the server 12 may store the tiled image of each hierarchy as illustrated in FIG. 6 for each focal length. Incidentally, the reason for changing the focal length is that, some of the specimen A10 may be translucent, and thus, has a focal length suitable for imaging the surface of the specimen A10 and a focal length suitable for imaging an inner portion of the specimen A10. In other words, by changing the focal length, the microscope 11 can generate a pathological image of the surface of the specimen A10 or a pathological image of an inner portion of the specimen A10.

In addition, a staining condition for the specimen A10 to be observed is another example of the imaging condition. Specifically, in pathological diagnosis, a specific portion (for example, a cell nucleus or the like) of the specimen A10 is stained using a fluorescent reagent in some cases. The fluorescent reagent is, for example, a substance that is excited and emits light when irradiated with light of a specific wavelength. In some cases, different luminous materials may be used to stain the same specimen A10. In this case, the server 12 may store a tiled image of each hierarchy as illustrated in FIG. 6 for each stained luminous material.

Furthermore, the number and resolution of the tiled images described above are merely examples, and can be appropriately changed depending on the system. For example, the number of tiled images combined by the server 12 is not limited to four. For example, the server 12 may repeat processing of combining 3 × 3 = 9 tiled images. Although the above is an example in which the resolution of the tiled image is 256 × 256, the tiled image may have resolution other than 256 × 256.

By using software adopting a system capable of handling the tiled image group having the hierarchical structure described above, the display control device 13 extracts a desired tiled image from the tiled image group having the hierarchical structure according to an input operation of the user via the display control device 13, and outputs the extracted tiled image to the display device 14. Specifically, the display device 14 displays an image of a certain part selected by the user among images of certain resolution selected by the user. With such processing, the user can obtain a feeling of observing the specimen while changing the observation magnification. That is, the display control device 13 functions as a virtual microscope. The virtual observation magnification here actually corresponds to the resolution.

However, the method of capturing a high-resolution image is not limited to the method of capturing the divided region while repeating the stop and move of the stage as described above, and any method may be used. For example, it is allowable to adopt an imaging method of imaging a divided region while moving the stage at a predetermined speed to acquire a high-resolution image on a strip, or the like. Furthermore, the processing of generating a tiled image from a high-resolution image is not essential, and it is also allowable to generate an image in which the resolution changes stepwise by causing the resolution of the entire high-resolution image combined with the stitching processing to change stepwise. Even in this case, it is possible to perform stepwise presentation of low-resolution images in a wide area range to high-resolution images in a small area to the user.

### [1-2-2. Viewing history information]

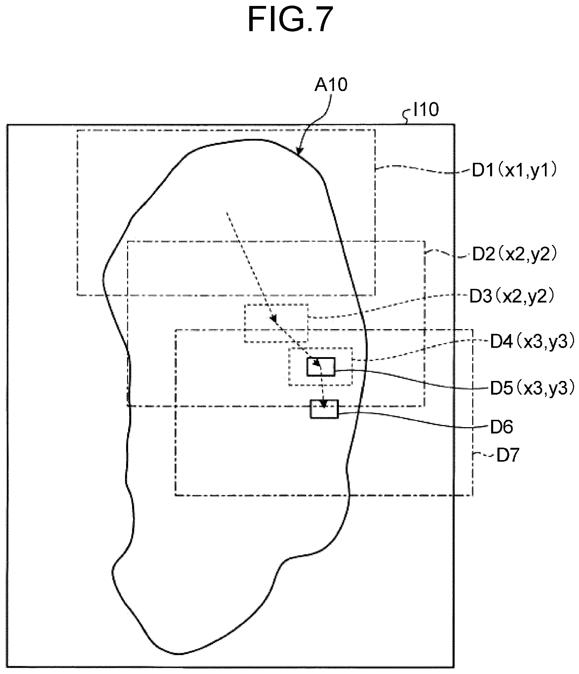

Next, the viewing history information of the pathological image stored in the server 12 or 22 will be described with reference to FIG. 7. FIG. 7 is a diagram illustrating an example of a viewing mode by a viewer of a pathological image. In the example illustrated in FIG. 7, it is assumed that a viewer such as a pathologist has viewed the regions D1, D2, D3,···, and D7 in this order in the pathological image I10. In this case, the display control device 13 first acquires the pathological image corresponding to the region D1 from the server 12 according to the viewing operation by the viewer. In response to a request from the display control device 13, the server 12 acquires one or more tiled images forming the pathological image corresponding to the region D1 from the storage unit, and transmits the acquired one or more tiled images to the display control device 13. Then, the display control device 13 displays, on the display device 14, the pathological image formed of one or more tiled images acquired from the server 12. For example, when there is a plurality of tiled images, the display control device 13 displays the plurality of tiled images adjacent to each other. Similarly, each time the viewer performs the operation of changing the display region, the display control device 13 acquires a pathological image corresponding to the region to be displayed (Regions D2, D3,···, D7, etc.) from the server 12 and displays the acquired pathological image on the display device 14.

In the example of FIG. 7, since the viewer first views a relatively wide region D1 and there is no region needing close observation in the region D1, the viewer moves the viewing region to the region D2. The viewer finds a region needing close observation in the region D2, and thus magnifies a partial region of the region D2 to view the region D3. Next, the viewer further moves to the region D4 which is a partial region of the region D2. Having found the region needing closer observation in the region D4, the viewer magnifies a partial region of the region D4 to view the region D5. In this manner, the viewer also views the regions D6 and D7. For example, the pathological images corresponding to the regions D1, D2, and D7 are display images at 1.25x magnification, the pathological images corresponding to the regions D3 and D4 are display images at 20x magnification, and the pathological images corresponding to the regions D5 and D6 are display images at 40x magnification. The display control device 13 acquires and displays the tiled image of the hierarchy corresponding to the each magnification in the tiled image group of the hierarchical structure stored in the server 12. For example, the tiled images corresponding to the regions D1 and D2 are at higher hierarchy (that is, a hierarchy close to the tiled image T1 illustrated in FIG. 6) than the hierarchy of tiled images corresponding to the region D3.

While the pathological image is viewed as described above, the display control device 13 acquires viewing information at a predetermined sampling period. Specifically, the display control device 13 acquires center coordinates and the display magnification of the viewed pathological image at each predetermined timing, and stores the acquired viewing information in the storage unit of the server 12.

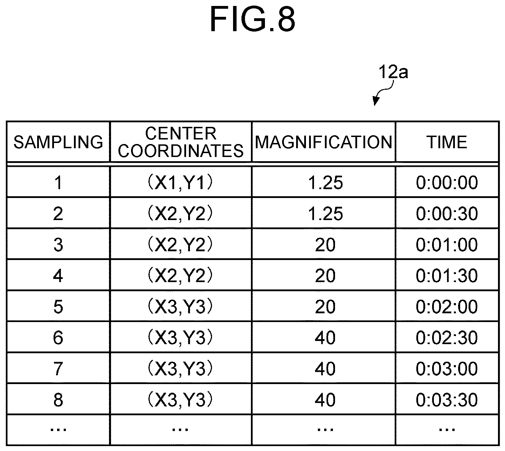

This point will be described with reference to FIG. 8. FIG. 8 is a diagram illustrating an example of a viewing history storage unit 12a included in the server 12. As illustrated in FIG. 8, the viewing history storage unit 12a stores information such as "sampling", "center coordinates", "magnification", and "time". "Sampling" indicates an order of viewing information storing timing. The "center coordinates" indicate position information of the viewed pathological image. In this example, the center coordinates are coordinates indicated by the center position of the viewed pathological image, and correspond to the coordinates of the coordinate system of the tiled image group in the lowermost layer. The "magnification" indicates a display magnification of the viewed pathological image. The "time" indicates an elapsed time from the start of viewing. The example of FIG. 8 illustrates that the sampling period is 30 seconds. That is, the display control device 13 stores the viewing information in the viewing history storage unit 12a every 30 seconds. However, the present invention is not limited to this example, and the sampling period may be, for example, 0.1 to 10 seconds, or may be the time outside this range.

In the example of FIG. 8, sampling "1" indicates viewing information regarding the region D1 illustrated in FIG. 7, sampling "2" indicates viewing information regarding the region D2, sampling "3" and "4" indicate viewing information regarding the region D3, sampling "5" indicates viewing information regarding the region D4, and sampling "6", "7", and "8" indicate viewing information regarding the region D5. That is, the example of FIG. 8 illustrates that the region D1 is viewed for about 30 seconds, the region D2 is viewed for about 30 seconds, the region D3 is viewed for about 60 seconds, the region D4 is viewed for about 30 seconds, and the region D5 is viewed for about 90 seconds. In this manner, the viewing time of each region can be extracted from the viewing history information.

Furthermore, the number of times each region has been viewed can be extracted from the viewing history information. For example, it is assumed that the number of times of display of each pixel of the displayed pathological image is increased by one each time of execution of a display region changing operation (for example, an operation of moving the display region or an operation of changing the display size). For example, in the example illustrated in FIG. 7, when the region D1 is first displayed, the number of times of display of each pixel included in the region D1 is one. Next, when the region D2 is displayed, the number of times of display of each pixel included in both the region D1 and the region D2 is two, while the number of times of display of each pixel included in the region D2 and not included in the region D1 is one. Since the display region can be specified by referring to the center coordinates and the magnification of the viewing history storage unit 12a, the number of times each pixel (which can also be referred to as each coordinate) of the pathological image is displayed can be extracted by analyzing the viewing history information stored in the viewing history storage unit 12a.

In a case where the operation of changing the display position has not been performed by the viewer for a predetermined time (for example, 5 minutes), the display control device 13 may suspend the viewing information storage processing. Furthermore, although the above is an example in which the viewed pathological image is stored as the viewing information by using the center coordinates and the magnification, the configuration is not limited to this example, and the viewing information may be any information as long as it can specify the region of the viewed pathological image. For example, the display control device 13 may store, as the viewing information of the pathological image, tile identification information for identifying the tiled image corresponding to the viewed pathological image or information indicating the position of the tiled image corresponding to the viewed pathological image. Furthermore, although not illustrated in FIG. 8, the viewing history storage unit 12a stores information for identifying a patient, a medical record, and the like. That is, the viewing history storage unit 12a illustrated in FIG. 8 stores the viewing information in association with the patient, the medical record, and the like.

### [1-3. Derivation device]

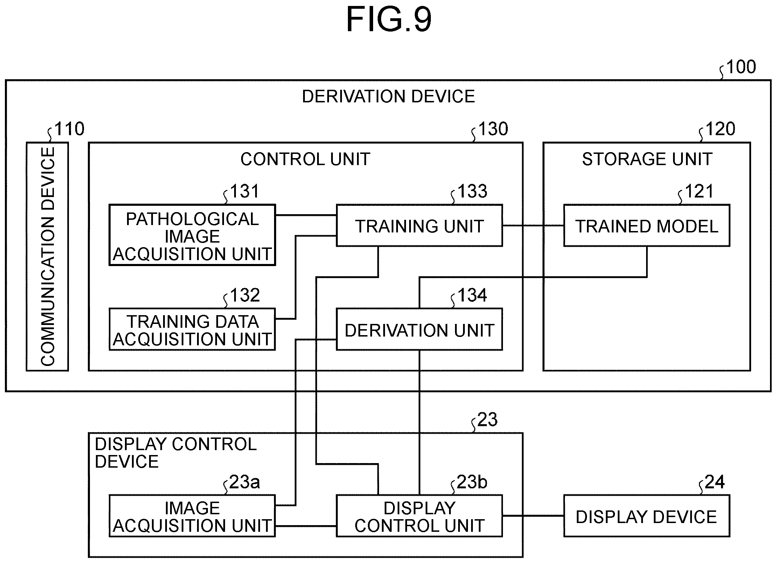

Next, the derivation device 100 according to the present embodiment will be described. Here, the display control device 23 will be described together with the derivation device 100. FIG. 9 is a diagram illustrating an example of a derivation device and a display control device according to the present embodiment. As illustrated in FIG. 9, the derivation device 100 is a computer including a communication unit 110, a storage unit 120, and a control unit 130.

The communication unit 110 is implemented by a network interface card (NIC), for example. The communication unit 110 is connected to a network (not illustrated) via a wired or wireless channel, and transmits and receives information to and from the pathology system 10, the pathology system 20, the medical information system 30, and the like via the network. The control unit 130 described below transmits and receives information to and from these devices via the communication unit 110.

The storage unit 120 is implemented by semiconductor memory elements such as random access memory (RAM) and flash memory, or other storage devices such as a hard disk or an optical disc. The storage unit 120 stores a trained model 121 created by the control unit 130. The trained model 121 will be described below.

The control unit 130 is implemented by execution of programs (diagnosis support program) stored inside the derivation device 100 by a central processing unit (CPU), a micro processing unit (MPU), or the like, using random access memory (RAM) or the like, as a working area. Furthermore, the control unit 130 may be executed by, for example, an integrated circuit such as an application specific integrated circuit (ASIC) or a field programmable gate array (FPGA).

As illustrated in FIG. 9, the control unit 130 includes a pathological image acquisition unit 131, a training data acquisition unit 132, a training unit 133, and a derivation unit 134, and implements or executes function and actions of information processing described below. The internal configuration of the control unit 130 is not limited to the configuration illustrated in FIG. 9, and may be any other configuration as long as it is a configuration that performs information processing described below.

The pathological image acquisition unit 131 acquires, via the communication unit 110, a pathological image, which is an example of training data used for training of the learning model performed by the training unit 133. Specifically, the pathological image acquisition unit 131 acquires a pathological image corresponding to the first affected tissue stored in the server 12 of the pathology system 10.

The training data acquisition unit 132 acquires, via the communication unit 110, an annotated pathological image, which is an example of training data used for training of the learning model performed by the training unit 133. Specifically, the training data acquisition unit 132 acquires, from the medical information system 30, an annotated pathological image of the first affected tissue corresponding to the pathological image.

The training unit 133 trains the learning model using the annotated pathological image acquired by the training data acquisition unit 132. With this training, the training unit 133 creates the trained model 121 for obtaining a diagnostic estimation result from the pathological image corresponding to the second affected tissue. The training unit 133 then stores the trained model 121 in the storage unit 120.

Note that, for example, weakly supervised learning can be applied to the training of the learning model by the training unit 133, and the following method can also be used.
"WILDCAT: Weakly Supervised Learning of Deep ConvNets for Image Classification, Pointwise Localization and Segmentation", CVPR 2017
(http://webia.lip6.fr/~durandt/pdfs/2017_CVPR/Durand_W ILDCAT_CVPR_2017.pdf)
"Attention-based Deep Multiple Instance Learning", 2018(https://arxiv.org/abs/1802.04712)

Note that the method of training the learning model by the training unit 133 may be based on any algorithm. For example, the training unit 133 can create the trained model 121 using various learning algorithms such as deep learning which is a machine learning method based on a multilayer deep neural network, support vector machine, clustering, and reinforcement learning.

Furthermore, the training unit 133 does not need to perform training of the learning model using all pathological images. For example, the training unit 133 may perform training of the learning model using a pathological image of particular interest. For example, it is allowable for the training unit 133 to perform, based on the viewing history information of each pathological image, training of the learning model using only a pathological image including a region viewed for a predetermined time or more, training of the learning model using only a pathological image including a region viewed at a predetermined magnification, or training using only a pathological image including a region viewed a predetermined number of times or more. Furthermore, for example, the training unit 133 may train the learning model using only the region viewed for a predetermined time or more, may train the learning model using only the region viewed at a predetermined magnification, or may train the learning model using only the region viewed a predetermined number of times or more. Furthermore, for example, the training unit 133 may determine that the center region of the pathological image is a region of interest, cut out only the center region of the pathological image to be used to perform training of the learning model.

The derivation unit 134 acquires the pathological image corresponding to the second affected tissue from the display control device 23 and inputs the acquired pathological image to the trained model 121 to cause the trained model 121 to derive a diagnostic estimation result based on the pathological image corresponding to the second affected tissue, and outputs the derived estimation result to a display control unit 23b.

### [1-4. Creating training data]

Next, creation of training data to be used for training of the trained model 121, stored in the storage unit 120 of the derivation device 100, will be described in detail with reference to the drawings.

### [1-4-1. Flow of creating training data]

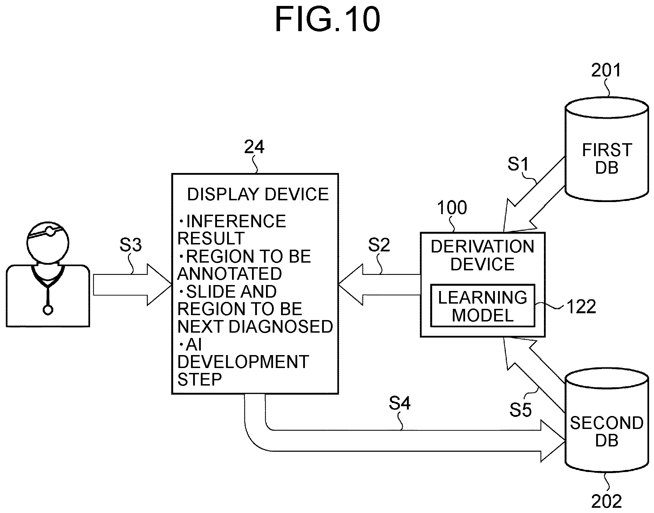

First, a flow of creating training data will be described in detail with reference to the drawings. FIG. 10 is a schematic diagram illustrating a flow when creating training data according to the present embodiment.

As illustrated in FIG. 10, in the present embodiment, the pathological image acquisition unit 131 in the control unit 130 of the derivation device 100 acquires, via the communication unit 110, a pathological image, which is an example of training data used for training of a learning model 122 performed by the training unit 133 (S1). Specifically, as described above, the pathological image acquisition unit 131 acquires, for example, a pathological image stored in a storage area (hereinafter, referred to as a first database 201) of the server 12 and/or 22 of the pathology system 10 and/or 20.

When having acquired the pathological image of the training target, the training unit 133 specifies a pathological image (that is, a pathological image to be recommended as a target to be annotated) important for training of the learning model 122 among the acquired pathological images, and specifies a region important for training of the learning model 122 in the specified pathological image. Subsequently, the training unit 133 causes the display device 24 to display the identified one or more pathological images and their regions via the display control unit 23b of the display control device 23 (S2). With this operation, the region to be annotated in each pathological image is recommended to the user.

Note that, S2 is step of displaying, on the display device 24, a pathological image to be annotated by the user and/or a region thereof, a label used for annotating a region of each pathological image, a current training stage (hereinafter, also referred to as a training step) of the learning model 122, and the like.

In addition, the display device 24 also displays an appropriate annotation class (hereinafter, also simply referred to as a class) as options for the user in order to support the user to appropriately annotate the region of each pathological image in each training step. Note that the annotation class is options of a diagnosis result to be used for annotating a region of each pathological image. Furthermore, the display device 24 also displays the pathological image and the regions thereof at an appropriate granularity, magnification, and the like for the above-described support.

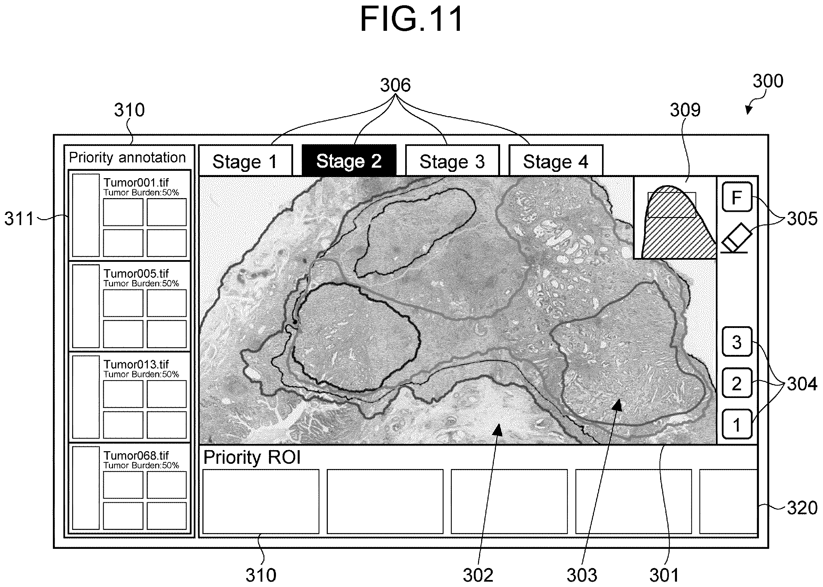

In response to such display, the user selects an annotation class to be applied to each region of the pathological image recommended by this display, for example, using a user interface (input unit) described below with reference to FIG. 11 (S3). This operation leads to creation of an annotated pathological image in which regions of the image are annotated.

The annotated pathological image created in this manner is accumulated in a second database 202 as training data. As described above, the second database 202 may be a storage area of the medical information system 30, for example. However, the present disclosure is not limited thereto, and it is allowable to appropriately change the area to the storage area of the server 12 and/or 22 of the pathology system 10 and/or 20, that is, the first database 201 or the like.

Thereafter, when a certain amount of training data has been accumulated in the second database 202, training of the learning model 122 is started. In training of the learning model 122, the training unit 133 of the derivation device 100 acquires the annotated pathological image that is training data from the second database 202 and trains the learning model 122 so as to create the trained model 121 (S5). The created trained model 121 is stored in the storage unit 120.

The operations of S1 to S5 described above are repeated, for example, until the trained model 121 with sufficient accuracy is created, resulting in updating or addition of the trained model 121 in the storage unit 120. This improves the accuracy of the trained model 121. However, the operation is not limited thereto, and for example, the operations of S1 to S5 may be executed each time a new pathological image is accumulated in the first database 201, periodically, or when an instruction by the user is given.

### [1-4-2. User interface]

Next, a user interface displayed on the display device 24 at creation of the training data will be described. FIG. 11 is a screen diagram illustrating an example of a user interface for creating training data according to the present embodiment.

As illustrated in FIG. 11, a user interface 300 for creating training data includes a next recommended image display region 310 and a priority ROI display region 320.

### (Pathological image display region 301)

A pathological image display region 301 displays a pathological image 302 selected by the user among the pathological images specified as the annotation target by the training unit 133. Furthermore, the pathological image 302 also displays a region 303 to be annotated. The region 303 is presented to the user, with a visual effect, as a region surrounded by a solid line, a broken line, or the like, or a region displayed with a contrast different from that of other regions (for example, regions needing no annotation), for example. Note that, when a plurality of regions 303 is included in one pathological image 302, each of the plurality of regions 303 may be visually distinguished by different colors, line types, contrasts, or the like.

Furthermore, for example, on the right-hand side of the pathological image display region 301, there is provided a class selection button 304 displayed for selecting how the region is to be annotated in order to enable the user to easily annotate the region. For example, the user selects the class selection button 304 in a state where one of the regions 303 is selected in the pathological image 302 displayed in the pathological image display region 301. This enables the selected region 303 to be annotated in the selected manner.

Note that, for example, it is allowable to display, on the right-hand side of the pathological image display region 301, an edit button 305 including an erase button for erasing the label used for annotating the region and an end button for ending the editing the label used for annotating the pathological image 302 and saving the annotated pathological image. Furthermore, the annotated region 303 may be distinguished from other regions 303 (non-annotated regions 303) by using visual effects such as different colors, line types, and high contrast, for example. Furthermore, the display of the visual effect (for example, lines, colors, highlights, and the like) of each region 303 displayed with superimposed display on the pathological image 302 may be independently turned on/off by an instruction of the user. This makes it possible to suppress occurrence of difficulty in diagnosing the region 303 as a target due to the visual effect of the other region 303 when annotating each of the plurality of regions 303 to be superimposed.

Furthermore, it is allowable to display, on the upper side of the pathological image display region 301, for example, a stage selection tag 306 for indicating the hierarchy (stage) of the pathological image 302 and the region 303 currently displayed in the pathological image display region 301 in the hierarchical structure of the pathological case described below and for switching the stage of the pathological image 302 to be annotated. FIG. 11 illustrates a state in which pathological cases are classified into a four-stage hierarchical structure of stage 1 to stage 4, and the pathological image 302 of stage 2 among the pathological cases is selected to be annotated. By switching the stage of the pathological image 302 displayed in the pathological image display region 301 by using the stage selection tag 306, the user can switch the pathological image 302 to be edited and can also confirm the hierarchical progress of diagnosis for each stage.

Furthermore, the pathological image display region 301 may be provided with an overhead region 309 that visually indicates which region in the entire image of the case the region displayed in the pathological image display region 301 corresponds to.

### (Next recommended image display region 310)

A next recommended image display region 310 displays a list of pathological images to be annotated next, for example, in descending order of the degree of recommendation. It is allowable to display, for each pathological image, for example, a thumbnail of the pathological image, a region to be annotated in the pathological image, a tumor content in the pathological image, or an identification class of each region predicted by the training unit 133.

### (Priority ROI display region 320)

A priority ROI display region 320 displays, for example, a list of regions to be annotated in the pathological image provisionally selected by the user in the next recommended image display region 310. Note that the provisional selection may represent, for example, a selection made by the user for one pathological image in the next recommended image display region 310 by clicking, touching, or the like. In this state, the information related to the selected pathological image may be displayed to be encircled by a thick frame or the like, for example. On the other hand, a final selection may be selecting the provisionally selected pathological image again by clicking, touching, or the like. With the final selection, the pathological image 302 displayed in the pathological image display region 301 may be switched to the pathological image determined by the final selection.

### [1-5. Creating trained model by multi-stage training step]

Next, creation of training data by a multi-stage training step will be described.

### [1-5-1. Hierarchical representation of pathological cases]

In general, it is known that a pathological case has a hierarchical structure. Clinically, slide data (pathological image) labels are usually assigned to the most malignant cases observed. For example, when a tumor such as an adenoma and cancer cells are observed in the large intestine, only the colorectal cancer is diagnosed. In addition, as Non Patent Literature 2, a plurality of pieces of diagnostic information as illustrated in Table 1 below can be collectively represented in four classes hierarchically. Furthermore, there are other methods of expressing pathological cases in a hierarchical class in various medical fields such as grade classification.

**Table 1**

| Diagnosis | Mapping |
|---|---|
| Non-proliferative Changes | Benign |
| Fibroadenoma (FA) | Benign |
| Intraductal Papilloma Without Atypia | Benign |
| Usual Ductal Hyperplasia(UDH) | Benign |
| Columnar Cell Hyperplasia/Columnar Cell Change | Benign |
| Sclerosing Adenosis | Benign |
| Dadial Scar/Complex Sclerosing Lesion | Benign |
| Flat Epithelial Atypia(FEA) | Atypia |
| Atypical Ductal Hyperplasia(ADH) | Atypia |
| Intraductal Papilloma With Atypia | Atypia |
| Atypical Lobular Hyperplasia(ALH) | Atypia |
| Ductal Carcinoma in-situ (DCIS) | DCIS |
| Lobular Carcinoma in-situ(LCIS) | DCIS |
| Invasive (ductal or tabular) cancer | Invasive |

### [1-5-2. Creating stepwise trained model]

Here, in an attempt to create a trained model capable of identifying a plurality of complicated cases (classes) at a time, there are problematic cases such as a case where the created trained model does not normally operate, and a case where annotation and training of a learning model takes lots of effort and time. In addition, when the identification target has a hierarchical structure including class classifications different for each layer as in the above-described pathological case, information of the hierarchical structure would be lost, leading to a possibility of significant deterioration of the identification performance for the case. In view of this, the present embodiment proposes a method of creating the trained model 121 in consideration of the hierarchical structure.

### [1-5-2-1. Stepwise creation flow]

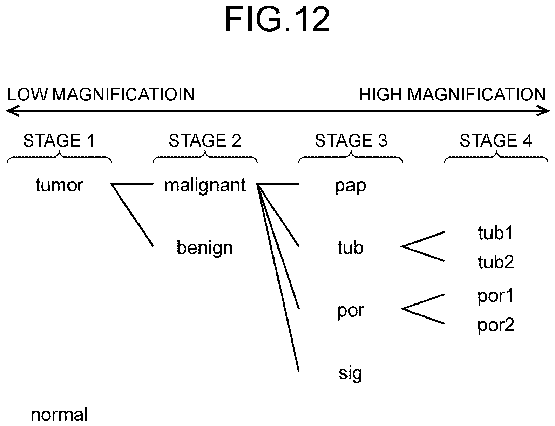

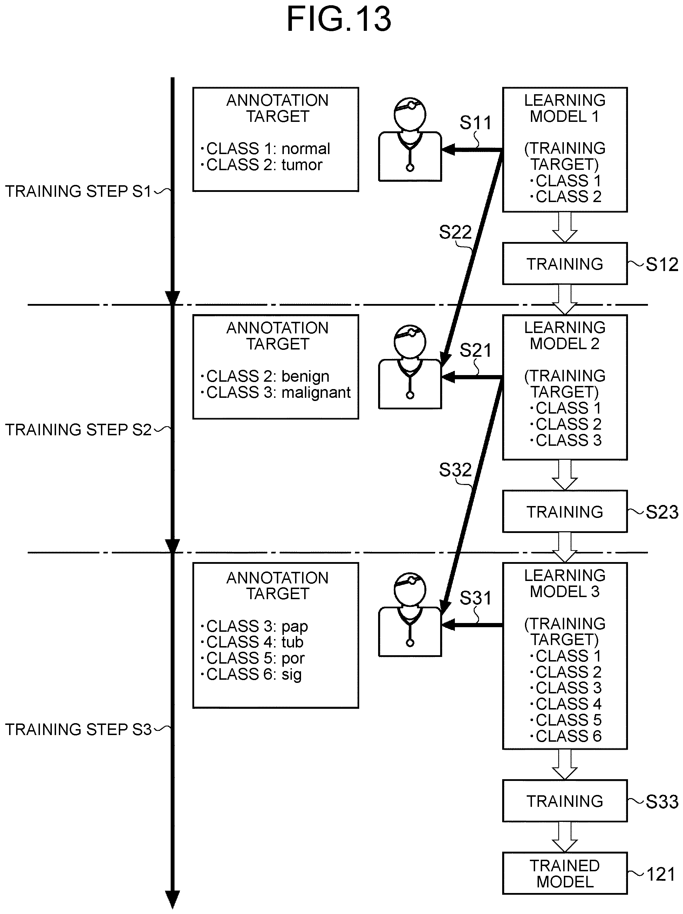

FIG. 12 is a hierarchical diagram illustrating an example of a hierarchical structure of a pathological case to be diagnosed. FIG. 13 is a schematic diagram illustrating an example of a stepwise trained model creation flow (hereinafter, referred to as an AI creation flow) according to the present embodiment.

As illustrated in FIG. 12, pathological cases can be roughly divided into two classes: tumor and normal (stage 1). Stage 1 tumors can be further divided into two classes: malignant and benign (stage 2). Furthermore, stage 2 malignant can be divided into four classes: papillary adenocarcinoma (pap), tubular adenocarcinoma (tub), poorly differentiated adenocarcinoma (por), and signet-ring cell carcinoma (sig) (stage 3). Furthermore, stage 3 tubular adenocarcinoma (tub) can be divided into two classes: well differentiated (tub 1) and moderately differentiated (tub 2), while poorly differentiated adenocarcinoma (por) can be divided into two classes: solid type (por1) and non-solid type (por2) (stage 4). In this manner, in the pathological case having the hierarchical structure, it is possible to set class classification in each layer. For example, the deeper the layer in the hierarchical structure, the more the number of class classifications.

In view of this, in the present embodiment, for example, the user selects a case (class) to be identified according to the hierarchical structure illustrated in FIG. 12. Moreover, in the present embodiment, an AI creation flow as illustrated in FIG. 13 is manually or automatically constructed. Note that, when the AI creation flow is manually constructed, the AI creation flow created by the user is set in the training unit 133 in the control unit 130 of the derivation device 100. At that time, manual construction of the AI creation flow may be supported by the training unit 133. On the other hand, when automatically constructing the AI creation flow, the training unit 133 automatically creates the AI creation flow according to the class to be identified set by the user, for example, by using a framework of the AI creation flow prepared in advance. Note that the hierarchical structure illustrated in FIG. 12 is merely an example, and the hierarchical structure of the case (class) may be manually or automatically created according to the class to be identified set by the user.

Note that, in FIG. 12, regarding the class of pathological cases, the higher the layer of the class of stage, the lower the magnification (for example, about 5x) for achieving identification on the pathological case, while the lower the layer of the class of stage, the higher magnification (for example, 20x to 40x, or more) would be needed for identification on the pathological image.

FIG. 13 illustrates an example of the AI creation flow when the class included in the stage 3 in FIG. 12 is selected as the identification target.

### (Training step S1)

The AI creation flow according to the present example first trains the learning model 122 (learning model 1 in FIG. 13) to learn the two highest (stage 1) classes (Class 1: normal, Class 2: tumor) in the hierarchical structure of the pathological case (training step S1).

Specifically, in the user interface 300 illustrated in FIG. 11, a region 303 inferred as normal and a region 303 inferred as tumor are superimposed (by recommendation of a region to be annotated) on the pathological image 302 displayed in the pathological image display region 301, thereby causing the user to execute an operation of annotating each region 303 with a label of normal or tumor (S11). Note that class the each region 303 may be inferred by various methods, for example, by the training unit 133 using the current trained model 121 or learning model 122 (for example, the learning model 1 before execution of the training step S1).

Subsequently, the training step S1 trains the learning model 122 using the pathological image (annotated pathological image) annotated with the two classes of stage 1 as training data (S12). With this operation, the trained model 121 (learning model 2 in FIG. 13) that has learned the two classes of stage 1 is created.

### (Training step S2)

The training step S2 trains the trained model 121 (learning model 2 in FIG. 13) to learn a total of three classes (Class 1: normal, Class 2: benign, Class 3: malignant) obtained as a result of classifying the Class 2 tumor of stage 1 into two classes (benign and malignant) in stage 2.

Specifically, in the user interface 300 illustrated in FIG. 11, a region 303 inferred as normal, a region 303 inferred as benign, and a region 303 inferred as malignant are superimposed (by recommendation of a region to be annotated) on the pathological image 302 displayed in the pathological image display region 301, thereby causing the user to execute an operation of annotating each region 303 with a label of normal, benign, or malignant (S21). Note that, similarly to training step S1, the class of each region 303 may be inferred by various methods, for example, by the training unit 133 using the current trained model 121 (for example, the learning model 2 before execution of the training step S2). Incidentally, the label used for annotating the region 303 in the training step S1 may be recommended to the user as a reference in the training step S2 (S22).

Subsequently, the training step S2 trains the trained model 121 using the pathological image (annotated pathological image) annotated with total of three classes of the stages 1 and 2 as training data (S23). With this operation, the trained model 121 (learning model 3 in FIG. 13) that has learned the total of three classes of the stages 1 to 2 is created.

### (Training step S3)

The training step S3, similarly to the training step S2, trains the trained model 121 (learning model 3 in FIG. 13) to learn a total of six classes (Class 1: normal, Class 2: benign, Class 3: papillary adenocarcinoma (pap), Class 4: tubular adenocarcinoma (tub), Class 5: poorly differentiated adenocarcinoma (por), Class 6: signet-ring cell carcinoma (sig)) obtained as a result of classifying the class 3 malignant of stage 2 into four classes (papillary adenocarcinoma (pap), tubular adenocarcinoma (tub), poorly differentiated adenocarcinoma (por), and signet-ring cell carcinoma (sig)) of stage 3.

Specifically, in the user interface 300 illustrated in FIG. 11, a region 303 inferred as normal, a region 303 inferred as benign, a region 303 inferred as papillary adenocarcinoma (pap), a region 303 inferred as tubular adenocarcinoma (tub), a region 303 inferred as poorly differentiated adenocarcinoma (por), and a region 303 inferred as signet-ring cell carcinoma (sig) are superimposed (by recommendation of a region to be annotated) on the pathological image 302 displayed in the pathological image display region 301, thereby causing the user to execute an operation of annotating each region 303 with a label of normal, benign, papillary adenocarcinoma (pap), tubular adenocarcinoma (tub), poorly differentiated adenocarcinoma (por), or sign-ring cell carcinoma (sig) (S31). Note that, similarly to training steps S1 and S2, the class of each region 303 may be inferred by various methods, for example, by the training unit 133 using the current trained model 121 (for example, the learning model 3 before execution of the training step S3). Incidentally, the label used for annotating the region 303 in the training step S2 may be recommended to the user as a reference in the training step S3 (S32).

Subsequently, in the training step S3, training of the trained model 121 is executed using the pathological image (annotated pathological image) annotated with total of six classes of the stages 1 and 3 as training data (S33). With this operation, the trained model 121 that has learned the total of six classes of the stages 1 to 3 is created.

Note that each of the training steps S1 to S3 may be executed a plurality of times. In that case, it is allowable to apply various flows such as a flow of executing each training step a plurality of times and then executing the next training step, or a flow of each training step is through training steps S1 to S3 and then executing again through training steps S1 to S3.

In this manner, by training the learning model stepwise to create the trained model 121, it is possible to present a region to be annotated to the user based on the previous training step information and next training step information, making it possible for user to appropriately annotate an appropriate region. In addition, since it is possible to effectively train the learning model in consideration of the hierarchical structure of the pathological case, achieving creation of the trained model 121 of each stage (training steps S1 to S3). Moreover, with a configuration that retains the trained model 121 created at each stage and that enables selective display of the identification result at each stage by the stage selection tag 306 in FIG. 11, it is possible to utilize the identification result of each training step with an ensemble effect.

### [1-6. Display with varied magnification and granularity]

Next, a function of recommending the magnification, the annotation granularity, and the like along the hierarchical structure of the pathological case to the user will be described. For example, when the region 303 is annotated with two types of labels of normal and tumor in the training step S1 of FIG. 13, it is also possible to provide the user with a function of roughly annotating the pathological image 302 as a low-magnification image in the low-layer training step. Furthermore, when a case is complicated in later training steps, it is also possible to provide the user with a function of finely annotating the pathological image 302 as a high-magnification image. Alternatively, it is also possible to change the granularity of annotation applied to the pathological image 302 not for each training step but according to the proficiency level of the learning model 122 or the trained model 121 in each training step.

In the present description, roughly annotating a pathological image of low magnification/high magnification is also referred to as application of low magnification/high magnification annotation to a pathological image. Furthermore, the magnification of the annotation applied on the pathological image is also referred to as granularity of annotation.

Hereinafter, a function of annotating with varied granularity (hereinafter, referred to as an annotation function) will be described with some examples.

### [1-6-1. First example]

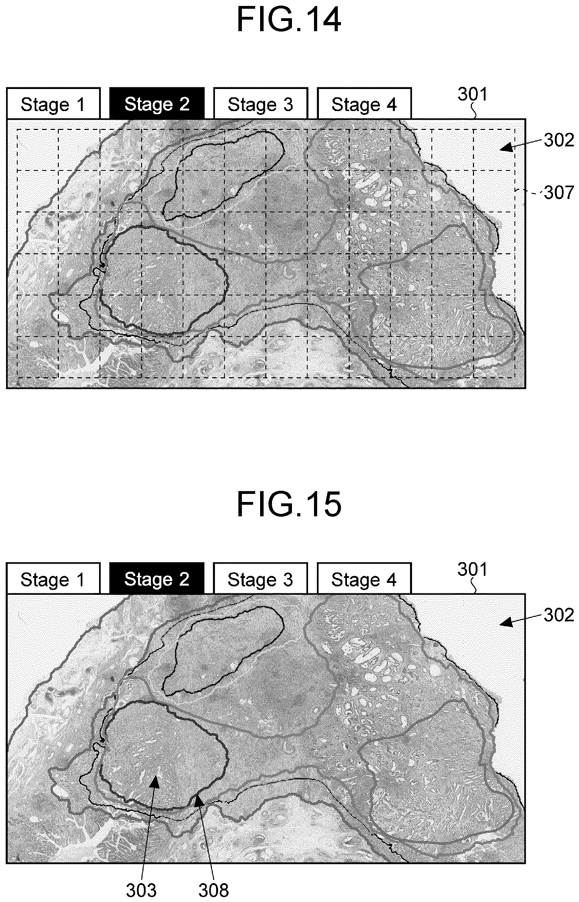

In a first example, an annotation function of roughly annotating a pathological image 302 as a low-magnification image will be described. FIG. 14 is a diagram illustrating the annotation function according to the first example. As illustrated in FIG. 14, in the annotation function according to the first example, a pathological image 302 as a low-magnification image is displayed in the pathological image display region 301 of the user interface 300 (refer to FIG. 11). In addition, the pathological image 302 displayed in the pathological image display region 301 also include a grid 307 displayed for the user to roughly set a region to be annotated. When the user selects the class selection button 304 while selecting one or more regions partitioned by the grid 307, the selected region is annotated.

### [1-6-2. Second example]

In a second example, another annotation function of roughly annotating the pathological image 302 as a low-magnification image will be described. FIG. 15 is a diagram illustrating an annotation function according to the second example. As illustrated in FIG. 15, in the annotation function according to the second example, a pathological image 302 as a low-magnification image is displayed in the pathological image display region 301 of the user interface 300 (refer to FIG. 11). The user can set the region 303 to be annotated by tracing the boundary of the region to be annotated in the pathological image 302 displayed in the pathological image display region 301 with a pointer 308 included in the user interface 300, for example, and can annotate the set region by selecting the class selection button 304 in a state where the region 303 is set.

### [1-6-3. Third example]

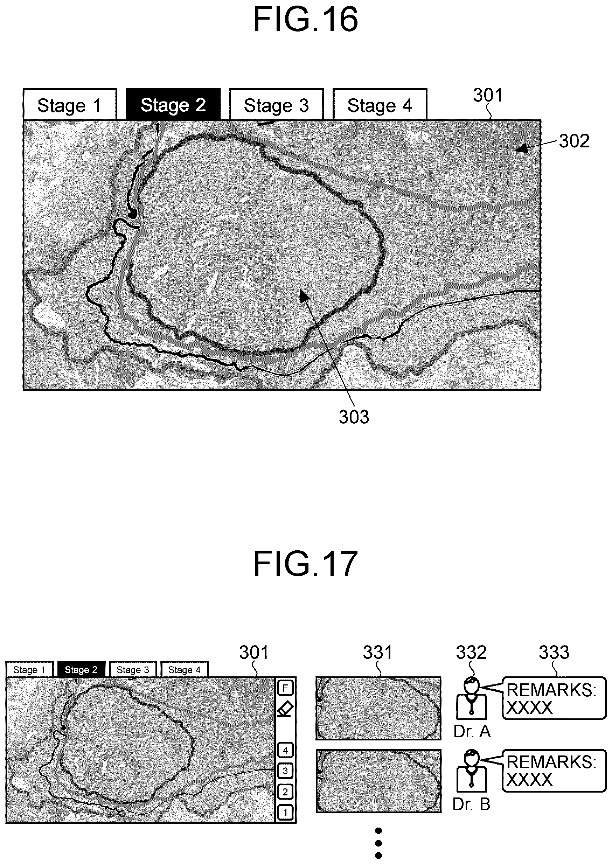

In a third example, an annotation function of finely annotating a pathological image 302 as a high-magnification image will be described. FIG. 16 is a diagram illustrating an annotation function according to a third example. As illustrated in FIG. 16, in the annotation function according to the third example, a pathological image 302 as a high-magnification image is displayed in the pathological image display region 301 of the user interface 300 (refer to FIG. 11). In addition, the pathological image 302 displayed in the pathological image display region 301 includes a region 303 displayed as an inferred region obtained using the trained model 121 trained in the training steps up to the previous stage. When the user selects the class selection button 304 while selecting the region 303, the selected region 303 is annotated.

### [1-7. Similarity search function with annotation]

Next, a similarity search function with annotation will be described. FIG. 17 is a diagram illustrating a similarity search function with annotation according to the present embodiment.

When the granularity of annotation becomes finer with the progress of the AI creation flow, the user can have a difficulty in deciding how the region 303 is to be annotated. Furthermore, at the initial stage of the AI creation flow, the user may have a difficulty in deciding to what level to annotate, such as blood vessels, stroma, and the like. In view of this, the present embodiment provides the user with a function (hereinafter, referred to as a similarity search function) of specifying a region of another pathological image similar to a region where the user has a difficulty in determination via the user interface 300.

The similarity search function also determines the similarity of the annotation applied on the region of the pathological image (a similarity search function with annotation) in addition to the similarity between the pathological images and/or between the regions. For example, when the similarity search function is used, a pathological image and/or region having a high similarity with a region difficult to be determined by the user and/or a pathological image including the region, is specified by a similar image search. Note that the similar image search may be a general similar image search. In addition, the similarity search function according to the present embodiment groups pathological images and/or the regions thereof having a higher degree of similarity, among the pathological images and/or the regions thereof specified by the similar image search, based on features used for annotating the pathological images and/or the regions by another user or the user in the past (hereinafter, referred to as a co-worker). Examples of the feature include the length of the labels for annotation, the area of the region, the number of labels for annotation, and the like.

As illustrated in FIG. 17, in a grouped result of the search, a pathological image 331 specified as a similar image, identification information (a name, a number, a photograph, an avatar, or the like) of a co-worker (a clinician, a pathologist, or the like) who has performed diagnosis (annotation) on the pathological image, and remarks 333 as diagnosis results included in a label used to annotate the pathological image are displayed in a region adjacent to the pathological image display region 301 in the user interface 300 in the example illustrated in FIG. 17.

In this manner, by grouping the results of the similarity search based on the annotation, it is possible to confirm the diagnosis details on the similar image by the co-worker, such as whether the blood vessel and the stroma included in the region of the tumor are carefully extracted or whether the annotation is roughly made as a mass. Furthermore, by displaying the comment such as the remarks 333 together with the pathological image 331, it is possible to use the comment as a reference at a time such as user's determination of the grade of the tumor.

### [1-8. Display example of inference result]

Next, a display example of the inference result obtained regarding the pathological image by using the trained model 121 as a multi-stage model created as described above will be described. As described above, the pathological image may be a pathological image corresponding to the second affected tissue to be diagnosed.

As described above, in the present embodiment, the trained model 121 for each hierarchy is created by training the learning model in multiple training steps (for example, training steps S1 to S3) according to the hierarchical structure of the pathological case. Accordingly, an inference result obtained by using the trained model 121 for each hierarchy also has a hierarchical structure. Therefore, in the present embodiment, the inference result having a hierarchical structure is presented to the user based on the hierarchical structure. This makes it possible to present the inference result of each hierarchy so as to be more easily interpreted by the user. Hereinafter, display of the inference result according to the hierarchical structure will be described with some examples.

### [1-8-1. First display example]

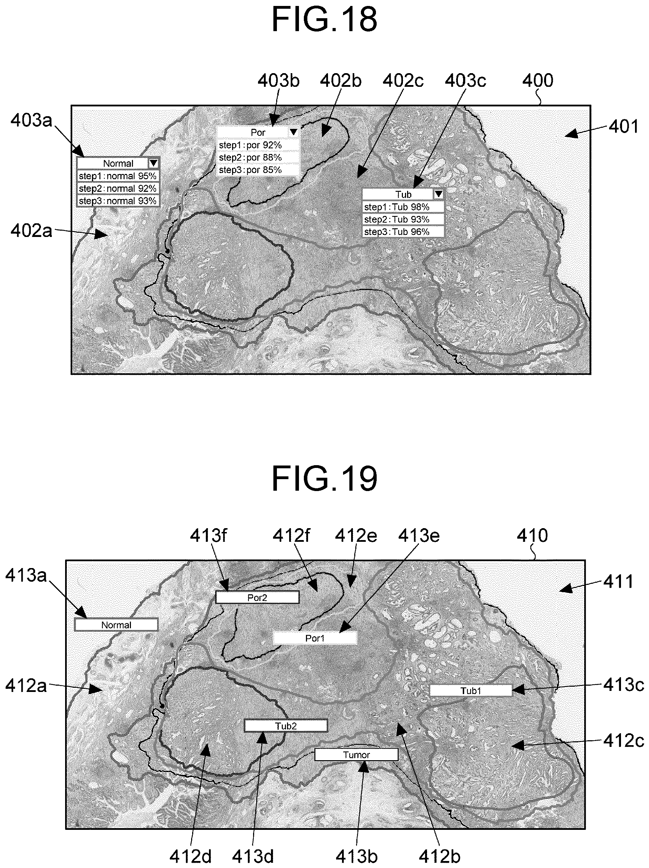

FIG. 18 is a diagram illustrating a user interface according to a first display example. As illustrated in FIG. 18, a user interface 400 according to the first display example displays a pathological image 401 to be diagnosed and annotation tags 403a to 403c indicating labels used for annotating each of regions 402a to 402c of the pathological image 401.

The top field of each of the annotation tags 403a to 403c displays a normal identification result. The normal identification result may be, for example, an inference result (class) obtained by using the final trained model 121.

Furthermore, each of the annotation tags 403a to 403c displays, with a pull-down method, for example, an inference result (hereinafter, referred to as an inference result of the training step) obtained by using the trained model 121 created in each training step (for example, training steps S1 to S3 illustrated in FIG. 13) and the reliability (for example, the probability that the annotation is correct) of the inference result. Note that the inference result of each training step may be an inference result with the highest reliability among the plurality of inference results. However, the display is not limited thereto, and the reliability calculated for each class in each training step may be displayed together with the annotation tags 403a to 403c.

In this manner, by displaying the inference result obtained by using the trained model 121 created in each training step together with the reliability of the inference result, it is possible to further enhance the user interpretation of a lesion included in the pathological image 401.

### [1-8-2. Second display example]

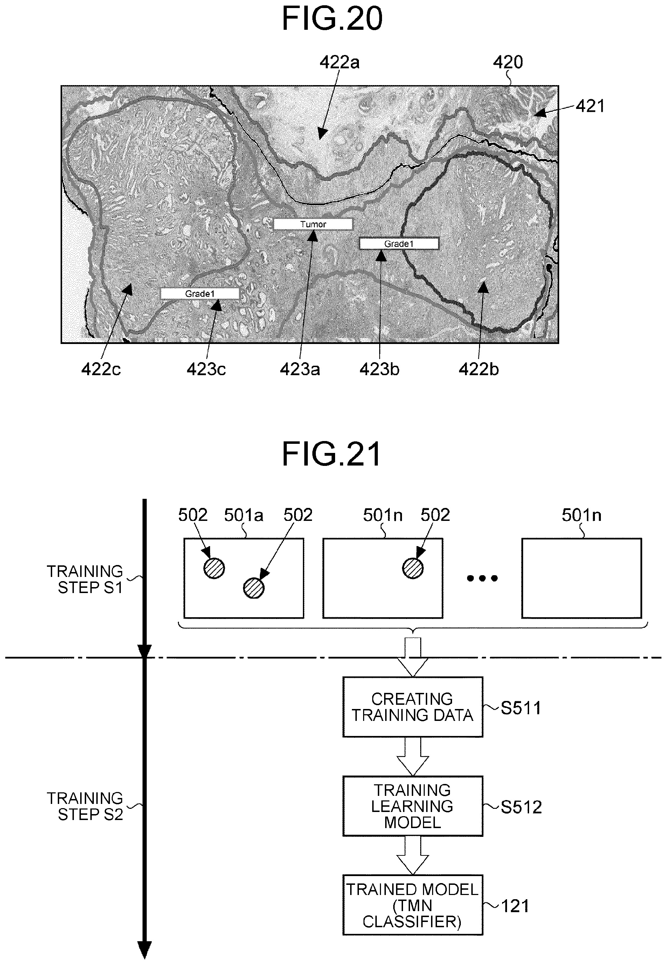

FIGS. 19 and 20 are diagrams illustrating a user interface according to a second display example.

First, in a user interface 410 according to the second display example illustrated in FIG. 19, all the regions 303 targeted in each training step are tagged with all the classes similarly targeted in each training step. Specifically, tags 412a and 412b of the class (normal and tumor) of stage 1 targeted in the training step S1 and tags 412c to 412f of the class (well differentiated (tub 1), moderately differentiated (tub 2), solid type (por1), non-solid type (por2)) of stage 4 targeted in the training step subsequent to the training step S3 not illustrated in FIG. 13 (referred to as the training step S4) are tagged to a pathological image 411 as a diagnosis target. In addition, although FIG. 19 does not include the class (benign and malignant) of the stage 2 targeted in the training step S2 or the class (papillary adenocarcinoma (pap), tubular adenocarcinoma (tub), poorly differentiated adenocarcinoma (por), and signet-ring cell carcinoma (sig)) of the stage 3 targeted in the training step S3, these classes are also tagged as necessary.

Furthermore, in the user interface 420 according to the second display example illustrated in FIG. 20, a tag 422a of a class (tumor) of stage 1 targeted in the training step S1 as well as tags 422b and 422c of classes (for example, Grade 1 and Grade 2) of other stages targeted in another training step not illustrated in FIG. 13 are tagged to the pathological image 421 as a diagnosis target.

In this manner, by displaying the inference results obtained by using the trained model 121 created in each training step together at the same time, it is possible for the user to trace the inference result output for each region 303, leading to further enhancement of user interpretation of each region 303 included in the pathological image 411. Note that, in FIGS. 19 and 20, a tag of a specific training step may be excluded from the display target based on setting by the user, for example.

### [1-9. Another example of hierarchical structure]

As another example of the hierarchical structure of the pathological case, there is a hierarchical structure of lymph node metastasis of breast cancer and the like, in addition to the hierarchical structure exemplified above with reference to FIG. 12. In the diagnosis for lymph node metastasis of breast cancer, whether or not there is a tumor, the size of the tumor, and the like are comprehensively determined based on a plurality of pathological images.

FIG. 21 is a diagram illustrating an example of an AI creation flow when the diagnosis support system 1 according to the present embodiment is applied to diagnosis for lymph node metastasis of breast cancer. As illustrated in FIG. 21, when the diagnosis support system 1 according to the present embodiment is applied to diagnosis for lymph node metastasis of breast cancer, the training step S1 of the AI creation flow detects a region 502 of a tumor in each of a plurality of pathological images 501a to 501n.

In the training step S2, training data is created by annotating the region detected in the training step S1 with a stage (corresponding to a class) based on the TMN classification (S511). Subsequently, training of the learning model 122 is executed using the created training data (S512). With this procedure, the trained model 121 as the TMN classifier is created.

Furthermore, the user interface configured to display the inference result obtained by using the trained model 121 created in this manner displays a plurality of target pathological images in parallel, while displaying a region of a tumor in each pathological image inferred by the trained model 121 and annotation (stage) applied to each region. By referring to these pieces of information (diagnosis grounds) displayed on the user interface, the user can give a final diagnosis result regarding lymph node metastasis of breast cancer.

### [1-10. Action and effects]

As described above, according to the present embodiment, it is possible to annotate a large number of pathological images stepwise. This makes it possible to reduce the amount of work and labor in creating training data, facilitating creation of a large amount of training data. This makes it possible to easily improve the accuracy of machine learning.

In addition, according to the present embodiment, it is possible to perform learning using stepwise application of annotation to a complicated case, leading to implementation of the diagnosis support system 1 capable of supporting diagnosis by a user in consideration of hierarchical information of the case.

Furthermore, by providing the appropriate granularity and magnification of the annotation according to the stage of learning, it is possible to apply annotation with higher efficiently.

Furthermore, identification results of the respective hierarchies can be displayed together when the trained model 121 is applied to actual diagnosis, making it possible provide diagnosis support information that can be easily interpreted by the user.

### (2. Other embodiments)

The processes according to each of embodiments described above may be performed in various different forms in addition to the configurations described above.

### [2-1. Display device]

The above embodiment illustrates an example in which diagnosis support UI screens (1) to (3) are displayed on the display device 24 being a stationary device. However, the diagnosis support information may be displayed on a body-worn device (a head-mounted display or the like) worn by a viewer of the pathological image displayed on the display device 24. At that time, diagnosis support information may be superimposed on the pathological image displayed on the display device 24. In addition, the diagnosis support information may be displayed on a transparent display attached so as to cover the front surface of the display device 24. At that time, the diagnosis support information may be displayed on the transparent display such that the diagnosis support information is superimposed on the pathological image displayed on the display device 24.

### [2-2. Imaging device]

Furthermore, although the above embodiment uses a microscope as an example of the device that images a specimen, the device is not limited thereto. For example, the device that images the specimen may be a medical image acquisition device for imaging the inside of a patient's body, such as an endoscope, computed tomography (CT), or magnetic resonance imaging (MRI). In this case, the server 12 and the server 22 store medical images such as twodimensional still images or moving images generated by an endoscope and three-dimensional images generated by CT or MRI. Furthermore, the server 12 and the server 22 may store information related to the image, such as an imaging condition and a diagnosis result for the image, in association with these images.

### [2-3. Server]

Furthermore, the server 12 and the server 22 may store other pathological images captured by other medical image acquisition devices such as an endoscope, CT, or MRI in association with the pathological image generated by the microscope. In this case, the display control unit 23b may display the other pathological images captured by the other imaging devices adjacent to each other for reference, in addition to the pathological image generated by the microscope.

### [2-4. Pathological image]

The pathological images stored in the server 12 and the server 22 include pathological images with low resolution. That is, there is a case where the resolution of the pathological image used as the training data is not the resolution enough to appropriately train the learning model. Here, when a glass slide containing a specimen is preserved, the glass slide may be re-imaged with a high-resolution microscope to newly generate a high-resolution pathological image. Accordingly, when the resolution of the pathological image used as the training data is not enough to perform appropriate training of the learning model and there is a re-imaged pathological image, the derivation device 100 may train the learning model using the re-imaged pathological image as training data.

### [2-5. Hardware configuration]

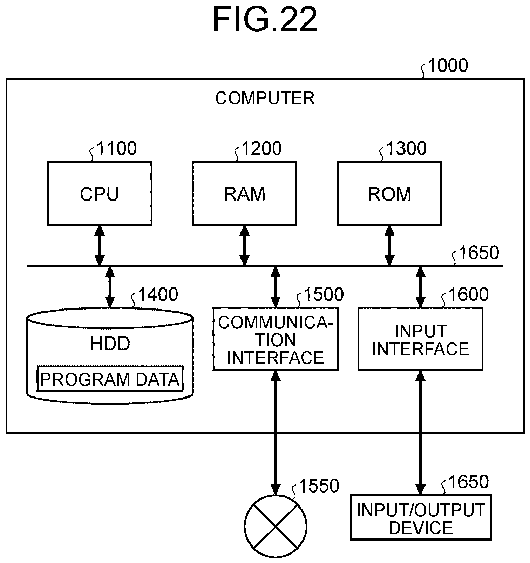

The information devices such as the derivation devices 100 and 200 and the display control device 23 according to the above-described embodiments are implemented by a computer 1000 having a configuration as illustrated in FIG. 22, for example. Hereinafter, the derivation device 100 according to the above-described embodiment will be described as an example. FIG. 22 is a hardware configuration diagram illustrating an example of a computer 1000 that implements functions of the derivation device 100. The computer 1000 includes a CPU 1100, RAM 1200, read only memory (ROM) 1300, a hard disk drive (HDD) 1400, a communication interface 1500, and an input/output interface 1600. Individual components of the computer 1000 are interconnected by a bus 1050.

The CPU 1100 operates based on a program stored in the ROM 1300 or the HDD 1400 so as to control each of components. For example, the CPU 1100 develops the program stored in the ROM 1300 or the HDD 1400 into the RAM 1200 and executes processing corresponding to various programs.

The ROM 1300 stores a boot program such as a basic input output system (BIOS) executed by the CPU 1100 when the computer 1000 starts up, a program dependent on hardware of the computer 1000, or the like.

The HDD 1400 is a non-transitory computer-readable recording medium that records a program executed by the CPU 1100, data used by the program, or the like. Specifically, the HDD 1400 is a recording medium that records a response generation program according to the present disclosure, which is an example of program data 1450.

The communication interface 1500 is an interface for connecting the computer 1000 to an external network 1550 (for example, the Internet). For example, the CPU 1100 receives data from other devices or transmits data generated by the CPU 1100 to other devices via the communication interface 1500.

The input/output interface 1600 is an interface for connecting between an input/output device 1650 and the computer 1000. For example, the CPU 1100 receives data from an input device such as a keyboard or a mouse via the input/output interface 1600. In addition, the CPU 1100 transmits data to an output device such as a display, a speaker, or a printer via the input/output interface 1600. Furthermore, the input/output interface 1600 may function as a media interface for reading a program or the like recorded on a predetermined computer-readable recording medium (or media). Examples of the media include optical recording media such as a digital versatile disc (DVD) or a phase change rewritable disk (PD), a magneto-optical recording medium such as a magneto-optical disk (MO), a tape medium, a magnetic recording medium, and semiconductor memory.

For example, when the computer 1000 functions as the derivation device 100 according to the above-described embodiment, the CPU 1100 of the computer 1000 executes the diagnosis support program loaded on the RAM 1200 to implement the functions of the pathological image acquisition unit 131, the training data acquisition unit 132, the training unit 133, the derivation unit 134, and the like. In addition, the HDD 1400 stores the diagnosis support program according to the present disclosure and data in the storage unit 120. Furthermore, for example, when the computer 1000 functions as the display control device 23 according to the above-described embodiment, the CPU 1100 of the computer 1000 executes the display control program loaded on the RAM 1200 to implement the functions of an image acquisition unit 23a, the display control unit 23b, and the like. In addition, the HDD 1400 stores a display control program according to the present disclosure. While the CPU 1100 executes program data 1450 read from the HDD 1400, the CPU 1100 may acquire the diagnosis support program and display control program from another device via the external network 1550, as another example.

### [Others]

Among individual processes described in the above embodiments, all or a part of the processes described as being performed automatically may be manually performed, or the processes described as being performed manually can be performed automatically by a known method. In addition, the processing procedures, specific names, and information including various data and parameters illustrated in the above Literatures or drawings can be arbitrarily altered unless otherwise specified. For example, various types of information illustrated in each of the drawings are not limited to the information illustrated.

In addition, each of components of each device is provided as a functional and conceptional illustration and thus does not necessarily need to be physically configured as illustrated. That is, the specific form of distribution/integration of each device is not limited to those illustrated in the drawings, and all or a part thereof may be functionally or physically distributed or integrated into arbitrary units according to various loads and use conditions.

Furthermore, the above-described embodiments and modifications can be appropriately combined within a range implementable without contradiction of processes.

The effects described in the present specification are merely examples, and thus, there may be other effects, not limited to the exemplified effects.

Note that the present technology can also have the following configurations.
(1) A determination support device comprising a derivation unit that derives an estimation result of determination on a first pathological image obtained by imaging a biological sample, the derivation being performed using a multi-stage trained model in which class classification can be set in each stage.
(2) The determination support device according to (1), further comprising a training unit that creates the multi-stage trained model by training a learning model using training data including annotations of different class classifications in each stage.
(3) The determination support device according to (2),
   wherein the multi-stage trained model includes: a first trained model; and a second trained model having the class classification different from the class classification of the first trained model, and
   the training unit performs processes including:
      creating first training data for creating the first trained model by annotating a region included in a second pathological image with one of classes of a first class classification;
      creating second training data for creating the second trained model by annotating the region included in the second pathological image with one of classes of a second class classification different in class classification from the first class classification;
      creating the first trained model by training the learning model using the first training data, and
      creating the second trained model by training the learning model using the second training data.
(4) The determination support device according to (3),
   wherein the training unit performs processes including:
   creating the first training data by annotating the second pathological image with the class selected by a user from the first class classification; and
   creating the second training data by annotating the second pathological image with the class selected by the user from the second class classification.
(5) The determination support device according to (4),
   wherein, in creating the second training data, the training unit presents, to the user, the estimation result of determination for the second pathological image estimated by the first trained model together with the second pathological image.
(6) The determination support device according to (4) or (5), wherein, in creating training data of each stage for creating each of the multi-stage trained models, the training unit presents, to the user, the estimation result of determination estimated by each of the multi-stage trained models together with the second pathological image.
(7) The determination support device according to any one of (3) to (6), wherein, in creating training data of each stage for creating each of the multi-stage trained models, the training unit presents, to the user, a grid for allowing a user to designate a region in the second pathological image together with the second pathological image.
(8) The determination support device according to any one of (3) to (6), wherein, in creating training data of each stage for creating each of the multi-stage trained models, the training unit causes a user to designate a region in the second pathological image, and annotates the designated region with the class designated by the user.
(9) The determination support device according to any one of (5) to (8), wherein, in creating training data of each stage for creating each of the multi-stage trained models, the training unit increases magnification of the second pathological image to be presented to the user with progress of the stage.
(10) The determination support device according to any one of (5) to (9), wherein, in creating training data of each stage for creating each of the multi-stage trained models, the training unit acquires one or more third pathological images similar to the second pathological image, and presents, to the user, information labeled to the one or more third pathological images and the one or more third pathological images together with the second pathological image.
(11) The determination support device according to (10), wherein the training unit preferentially presents, to the user, a third pathological image, out of the one or more third pathological images, the third pathological image being annotated with a label similar to a label recommended to be used for annotating the region of the second pathological image.
(12) The determination support device according to any one of (1) to (12), further comprising a display control unit that causes a display device to display the estimation result of determination derived by each of the multi-stage trained models.
(13) The determination support device according to (12), wherein the display control unit causes the display device to display the estimation result of determination derived in each of the multi-stage trained models together with reliability of each of the estimation results.
(14) The determination support device according to (12), wherein the display control unit causes the display device to display the estimation result of determination derived in each of the multi-stage trained models so as to be superimposed on the same first pathological image.
(15) The determination support device according to any one of (2) to (11), wherein the trained model creates the multi-stage trained model by training the learning model by deep learning using a multi-layer neural network.
(16) An information processing device for creating a multi-stage trained model that derives an estimation result of determination from a first pathological image obtained by imaging a biological sample,
   the information processing device comprising a training unit that creates the multi-stage trained model by training a learning model using training data including labels for annotation indicating different class classifications in each stage.
(17) A training method for creating a multi-stage trained model that derives an estimation result of determination from a first pathological image obtained by imaging a biological sample,
   the training method comprising creating the multi-stage trained model by training a learning model using training data including labels for annotation indicating different class classifications in each stage.

### Reference Signs List

- 1: DIAGNOSIS SUPPORT SYSTEM
- 10, 20: PATHOLOGY SYSTEM
- 11, 21: MICROSCOPE
- 12, 22: SERVER
- 13, 23: DISPLAY CONTROL DEVICE
- 14, 24: DISPLAY DEVICE
- 23a: IMAGE ACQUISITION UNIT
- 23b: DISPLAY CONTROL UNIT
- 30: MEDICAL INFORMATION SYSTEM
- 100: DERIVATION DEVICE
- 110: COMMUNICATION UNIT
- 120: STORAGE UNIT
- 121: TRAINED MODEL
- 122: LEARNING MODEL
- 130: CONTROL UNIT
- 131: PATHOLOGICAL IMAGE ACQUISITION UNIT
- 132: TRAINING DATA ACQUISITION UNIT
- 133: TRAINING UNIT
- 134: DERIVATION UNIT
- 201: FIRST DATABASE
- 202: SECOND DATABASE
- 300: USER INTERFACE

## Claims

1. A determination support device comprising a derivation unit that derives an estimation result of determination on a first pathological image obtained by imaging a biological sample, the derivation being performed using a multi-stage trained model in which class classification can be set in each stage.

2. The determination support device according to claim 1, further comprising a training unit that creates the multi-stage trained model by training a learning model using training data including annotations of different class classifications in each stage.

3. The determination support device according to claim 2,
wherein the multi-stage trained model includes: a first trained model; and a second trained model having the class classification different from the class classification of the first trained model, and
the training unit performs processes including:
creating first training data for creating the first trained model by annotating a region included in a second pathological image with one of classes of a first class classification;
creating second training data for creating the second trained model by annotating the region included in the second pathological image with one of classes of a second class classification different in class classification from the first class classification;
creating the first trained model by training the learning model using the first training data, and
creating the second trained model by training the learning model using the second training data.

4. The determination support device according to claim 3,
wherein the training unit performs processes including:
creating the first training data by annotating the second pathological image with the class selected by a user from the first class classification; and
creating the second training data by annotating the second pathological image with the class selected by the user from the second class classification.

5. The determination support device according to claim 4,
wherein, in creating the second training data, the training unit presents, to the user, the estimation result of determination for the second pathological image estimated by the first trained model together with the second pathological image.

6. The determination support device according to claim 4, wherein, in creating training data of each stage for creating each of the multi-stage trained models, the training unit presents, to the user, the estimation result of determination estimated by each of the multi-stage trained models together with the second pathological image.

7. The determination support device according to claim 3, wherein, in creating training data of each stage for creating each of the multi-stage trained models, the training unit presents, to the user, a grid for allowing a user to designate a region in the second pathological image together with the second pathological image.

8. The determination support device according to claim 3, wherein, in creating training data of each stage for creating each of the multi-stage trained models, the training unit causes a user to designate a region in the second pathological image, and annotates the designated region with the class designated by the user.

9. The determination support device according to claim 5, wherein, in creating training data of each stage for creating each of the multi-stage trained models, the training unit increases magnification of the second pathological image to be presented to the user with progress of the stage.

10. The determination support device according to claim 5, wherein, in creating training data of each stage for creating each of the multi-stage trained models, the training unit acquires one or more third pathological images similar to the second pathological image, and presents, to the user, information labeled to the one or more third pathological images and the one or more third pathological images together with the second pathological image.

11. The determination support device according to claim 10, wherein the training unit preferentially presents, to the user, a third pathological image, out of the one or more third pathological images, the third pathological image being annotated with a label similar to a label recommended to be used for annotating the region of the second pathological image.

12. The determination support device according to claim 1, further comprising a display control unit that causes a display device to display the estimation result of determination derived by each of the multi-stage trained models.

13. The determination support device according to claim 12, wherein the display control unit causes the display device to display the estimation result of determination derived in each of the multi-stage trained models together with reliability of each of the estimation results.

14. The determination support device according to claim 12, wherein the display control unit causes the display device to display the estimation result of determination derived in each of the multi-stage trained models so as to be superimposed on the same first pathological image.

15. The determination support device according to claim 2, wherein the trained model creates the multi-stage trained model by training the learning model by deep learning using a multi-layer neural network.

16. An information processing device for creating a multi-stage trained model that derives an estimation result of determination from a first pathological image obtained by imaging a biological sample,
the information processing device comprising a training unit that creates the multi-stage trained model by training a learning model using training data including labels for annotation indicating different class classifications in each stage.

17. A training method for creating a multi-stage trained model that derives an estimation result of determination from a first pathological image obtained by imaging a biological sample,
the training method comprising creating the multi-stage trained model by training a learning model using training data including labels for annotation indicating different class classifications in each stage.
